# EUROPEAN PATENT SPECIFICATION

(11) **EP 1 610 719 B1**
(45) Date of publication and mention of the grant of the patent: **13.01.2010**
(21) Application number: 04749472.9
(22) Date of filing: 26.03.2004
(51) Int. Cl.: A61F 2/04, A61F 5/00

(54) **SLEEVE FOR DELAYED INTRODUCTION OF ENZYMES INTO THE INTESTINE**
SCHLAUCH ZUR VERZÖGERTEN EINLEITUNG VON ENZYMEN IN DEN DARM
MANCHON POUR ENZYMES

(30) Priority: 28.03.2003 US 459060 P
(43) Date of publication of application: 04.01.2006
(73) Proprietor: GI Dynamics, Inc., Lexington MA 02421 (US)
(72) Inventor: LEVINE, Andy, H., Newton, MA 02461 (US); MEADE, John, C., Mendon, MA 01756 (US)
(74) Representative: Jenkins, Peter David
(86) International application number: PCT/US2004/009448
(87) International publication number: WO 2004/087233

(56) References cited:
- US-A- 5 306 300
- US-A- 5 820 584
- US-A1- 2003 040 804
- US-A1- 2003 040 808
- US-A1- 2003 109 931

## Description

### BACKGROUND OF THE INVENTION

According to the Center for Disease Control (CDC), over sixty percent of the United States population is overweight, and almost twenty percent are obese. This translates into 38.8 million adults in the United States with a Body Mass Index (BMI) of 30 or above. The BMI is defined as a person's weight (in kilograms) divided by height (in meters), squared. To be considered clinically, morbidly obese, one must meet at least one of three criteria: (i) BMI over 35; (ii) 45 kg (100 pounds) overweight; or (iii) 100% above an "ideal" body weight. There is also a category for the super-obese for those weighing over 159 kg (350 pounds).

Obesity is an overwhelming health problem. Because of the enormous strain associated with carrying this excess weight, organs are affected, as are the nervous and circulatory systems. In 2000, the National Institute of Diabetes, Digestive and Kidney Diseases (NIDDK) estimated that there were 280,000 deaths directly related to obesity. The NIDDK further estimated that the direct cost of healthcare in the US. associated with obesity is $51 billion. In addition, Americans spend $33 billion per year on weight loss products. In spite of this economic cost and consumer commitment, the prevalence of obesity continues to rise at alarming rates. From 1991 to 2000, obesity in the US. grew by 61%. Not exclusively a US. problem, worldwide obesity ranges are also increasing dramatically.

One of the principal costs to the healthcare system stems from the co- morbidities associated with obesity. Type-2 diabetes has climbed to 7.3% of the population. Of those persons with Type-2 diabetes, almost half are clinically obese, and two thirds are approaching obese. Other co-morbidities include hypertension, coronary artery disease, hypercholesteremia, sleep apnea, and pulmonary hypertension.

Although the physiology and psychology of obesity are complex, the medical consensus is that the cause is quite simple -- an over intake of calories combined with a reduction in energy expenditures seen in modern society. While the treatment seems quite intuitive, the institution of a cure is a complex issue that has so far vexed the best efforts of medical science. Dieting is not an adequate long-term solution for most people. Once an individual has slipped past the BMI of 30, significant changes in lifestyle are the only solution.

There have been many attempts in the past to surgically modify patients' anatomies to attack the consumption problem by reducing the desire or ability to eat. Stomach staples, or gastroplasties, to reduce the volumetric size of the stomach, therein achieving faster satiety, were performed in the 1980's and early 1990's. Although able to achieve early weight loss, sustained reduction was not obtained. The reasons are not all known, but are believed related to several factors. One of which is that the stomach stretches over time increasing volume, while psychological drivers motivate patients to find creative approaches to literally eat around the smaller pouch.

There are currently two surgical procedures that successfully produce long-term weight loss: the Roux-en-Y gastric bypass; and the biliopancreatic diversion with duodenal switch (BPD). Both procedures reduce the size of the stomach plus shorten the effective-length of intestine available for nutrient absorption. Reduction of the stomach size reduces stomach capacity and the ability of the patient to take in food. Bypassing the duodenum makes it more difficult to digest fats, high sugar, and carbohydrate rich foods. One objective of the surgery is to provide feedback to the patient by producing a dumping syndrome if they do eat these food products. Dumping occurs when carbohydrates directly enter the jejunum without being first conditioned in the duodenum. The result is that a large quantity of fluid is discharged into the food from the intestinal lining. The total effect makes the patient feel light-headed and results in severe diarrhea. For reasons that have not been determined the procedure also has an immediate therapeutic effect on diabetes.

Although the physiology seems simple, the exact mechanism of action in these procedures is not understood. Current theory is that negative feedback is provided from both regurgitation into the oesophagus and dumping when large volumes of the wrong foods are eaten. Eventually, patients learn that to avoid both these issues they must be compliant with the dietary restrictions imposed by their modified anatomy. In the BPD procedure, large lengths of jejunum are bypassed resulting in malabsorption and therefore, reduced caloric uptake. In fact, the stomach is not reduced in size as munch in the BPD procedure so that the patient is able to consume sufficient quantities of food to compensate for the reduced absorption. This procedure is reserved for the most morbidly obese as there are several serious side effects of prolonged malabsorption.

Unfortunately, these procedures carry a heavy toll. The morbidity rate for surgical procedures is alarmingly high with 11% requiring surgical intervention for correction. Early small bowel obstruction occurs at a rate of between 2 to 6% in these surgeries and mortality rates are reported to be approximately 0.5 to 1.5%. While surgery seems to be an effective answer, the current invasive procedures are not acceptable with these complication rates. Laparoscopic techniques applied to these surgeries provide faster recovery but continue to expose these very ill patients to high operative risk in addition to requiring an enormous level of skill by the surgeon. Devices to reduce absorption in the small intestines have been proposed (See US. Patent Number 5,820, 584 (Crabb), US. Patent Number 5,306, 300 (Berry) and US. Patent Number 4,315,509 (Smit)). However, these devices have not been successfully implemented.

US-A-5,306,300 discloses a tubular digestive screen having a tube with a firm ring at the top of the tube.

### SUMMARY OF THE INVENTION

The present invention provides a gastrointestinal implant device comprising: an elongated tube defining a central lumen, the tube being open at both ends, and adapted to extend into the duodenum; and an anchor for coupling the proximal end of the tube in alignment with the hepatopancreatic ampulla, wherein the anchor is adapted to be positioned within the hepatopancreatic ampulla, the tube being able to pass digestive enzymes from the hepatopancreatic ampulla into a distal portion of the gastrointestinal tract.

One means of reducing caloric uptake is to reduce the ability of the body to both breakdown the foods and to incorporate the foods. Normally, partially-digested food, or chyme, enters the duodenum from the stomach and mixes with enzymes introduced through the hepatopancreatic ampulla. The invention relates to a gastrointestinal implant including an elongated tube, open at both ends, that takes digestive enzymes from the point at which they enter the duodenum, and deposits them downstream, such as into the distal jejunum several feet down. This delays the mixing of the enzymes with the chyme and thus, the breakdown and subsequent digestion of food. The proximal end of the elongated tube can be secured in alignment with the hepatopancreatic ampulla, below the common bile duct. The tube can include a flexible sleeve portion attached to an anchor and then draped distally, through the intestines. The anchor, for example, can be a stent-like device. Natural peristalsis carries the enzymes through the tube. The enzymes then exit the tube and mix with the chyme further downstream than is normal.

The flexible sleeve is formed of a flexible material, such as TEFLON® film (e.g. polytetrafluoroethylene (PTFE), or Fluorinated Ethylene Polymer (FEP), or combinations thereof), expanded PTFE (ePTFE), polypropylene and/or polyethylene. Additionally, the flexible sleeve can be coated with one or more materials having the same or different properties than the underlying sleeve material. For example, a polyurethane and/or silicone coating can be applied to the flexible sleeve. Preferably, the sleeve material and/or its coating provides a relatively low coefficient of friction. For example, the sleeve material and/or coating can have a coefficient of friction of less than about 0, 2.

The anchor is typically formed as a cylinder, generally defining a lumen along its central axis. The external diameter of the anchor can be selected according to the anatomy of the patient. The external diameter, of the anchor is selected to provide an interference fit when inserted into the hepatopancreatic ampulla. For example, the anchor can have an external diameter between about 5 and about 10 millimeters (mm). More preferably, the anchor can have an external diameter between about 8 and about 10 mm. Additionally, the anchor has a length that is sufficiently long to facilitate securing it to the body, but not so long that it extends beyond the point where the pancreatic duct and the common bile duct join. Such a configuration ensures that both bile from the common bile duct and pancreatic enzymes from the pancreatic duct are collected into the sleeve. For example, the anchor can have an overall length between about 1 and about 5 centimeters (cm).

In some embodiments, the anchor is collapsible, facilitating its implantation and removal. For example, the anchor can be fashioned from a shape memory material, such as a nickel-titanium (Ni-Ti) alloy. One such Ni-Ti alloy is commonly referred to as nitinol. In other embodiments, the anchor can include a stent formed from a rigid, semi-rigid, or flexible material

The anchor is generally attached to the proximal end of the sleeve. For example, the anchor can be bonded, and/or molded to the sleeve. In some embodiments, the anchor is covered along its interior and exterior surfaces by a proximal portion of the flexible sleeve. Further, the anchor can include mechanical fasteners, such as barbs extending from the exterior surface of the anchor to secure the proximal portion of the sleeve to the hepatopancreatic ampulla. The barbs are preferably configured to secure the proximal end of the flexible sleeve, anchoring it into bodily tissue. Further, the barbs can be bi-directional, extending outward, in opposing directions that are substantially parallel to the central axis of the proximal end of the flexible sleeve.

In other embodiments, the gastrointestinal implant device is secured to the body using a two-piece anchor including a non-removable element, or fixed anchor, and a removable element. The fixed anchor can be secured in the hepatopancreatic ampulla. The removable element, attached to the proximal end of the sleeve, is configured for removable attachment to the fixed anchor. For example, the fixed anchor can contain a feature, such as a rim. The removable element can include complementary feature configured for engaging the fixed anchor. For example, the removable element can include a lip configured to engage the rim of the fixed anchor.

Alternatively, or in addition, the anchor includes an annular element having retractable staples. The staples, when engaged, couple the proximal end of the anchor to bodily tissue. The anchor can also be fastened using sutures, an adhesive, or any other suitable means of attachment.

The device of the invention can be used in a process for enabling weight loss. The process includes the steps of providing an elongated flexible sleeve open at both ends, the sleeve being adapted to extend into the duodenum. Further, the process includes aligning a proximal end of the flexible sleeve to the hepatopancreatic ampulla, and anchoring the proximal end to the hepatopancreatic ampulla. The process then includes extending the distal end of the sleeve into a distal portion of the gastrointestinal tract such that the elongated flexible sleeve deposits digestive enzymes via peristalsis directly into the distal intestine, such as the distal jejunum or the ileum.

Still further, the device of the invention can be used in a process for treating Type-2 diabetes. The process includes the steps of providing an elongated flexible sleeve, open at both ends, and adapted to extend into the duodenum. The proximal end of the flexible sleeve is aligned with the hepatopancreatic ampulla and anchored thereto. Further, the distal end of the sleeve is extended into a distal portion of the gastrointestinal tract such that the elongated flexible sleeve deposits digestive enzymes via peristalsis directly into the distal intestine.

### BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.
FIG.1 is a sectional view of a portion of the gastrointestinal tract of an animal body containing an exemplary embodiment of an enzyme sleeve implanted therein;
FIG. 2 is a sectional view of a portion of the gastrointestinal tract illustrating in more detail the enzyme sleeve shown in FIG.1;
FIGS. 3A and 3B are schematic diagrams illustrating a side projection view of one embodiment of a tubular anchor respectively shown in an expanded configuration and in a contracted configuration;
FIG. 4A provides top and side orthonormal projection views of an alternative embodiment of a tubular anchor;
FIG. 4B is a perspective view of the tubular anchor shown in FIG.4A;
FIG. 5 is a schematic diagram of a portion of the gastrointestinal tract illustrating an exemplary anchoring means for securing a proximal end of the enzyme sleeve below the common bile duct;
FIG. 6 is a schematic diagram of a portion of the gastrointestinal tract illustrating an alternative anchoring means for securing the proximal end of the enzyme sleeve below the common bile duct; and
FIG. 7 is a schematic diagram of a portion of the gastrointestinal tract illustrating an alternative, two-piece anchoring means for securing the proximal end of the enzyme sleeve below the common bile duct.

### DETAILED DESCRIPTION OF THE INVENTION

A description of preferred embodiments of the invention follows.

One means of reducing caloric uptake is to reduce the ability of the body to both breakdown the foods and to incorporate the foods. The invention relates to a gastrointestinal implant including a flexible, collapsible tube, open at both ends, that takes digestive enzymes from the point at which they enter the duodenum, and deposits them into the distal jejunum, or ileum, several feet down. This delays the breakdown and subsequent digestion of food. The proximal end of the tube can be secured in the hepatopancreatic ampulla, below the common bile duct with an anchor, such as a stent-like device. A sleeve portion of the tube is attached to the anchor and then draped distally, through the intestines. Natural peristalsis carries the enzymes through the tube distally. The enzymes then exit the tube and mix with the chyme further down stream than is normal.

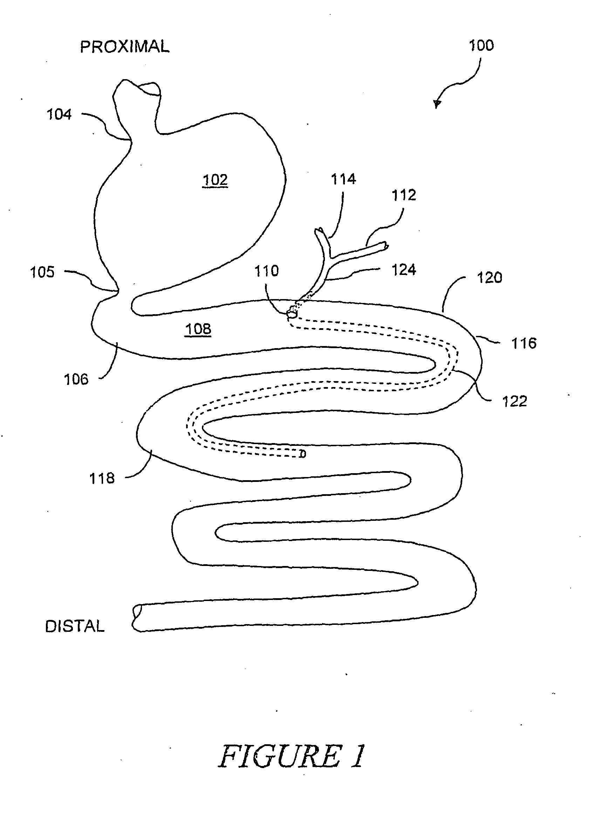

FIG.1 is a sectional view of a portion of the digestive tract 100 of an animal body. Food to be digested enters the stomach 102 through the cardiac orifice 104 from the oesophagus. Chyme, a semi-fluid, homogeneous creamy or gruel-like material produced by gastric digestion in the stomach exits the stomach through the pyloric orifice (pylorus) 105 and enters the small intestine 106. The pylorus 105 is a distal aperture of the stomach 102 surrounded by a strong band of circular muscle. The small intestine 106 in an average human body is a convoluted tube, about 4.6 m (15 feet) in length, extending from the pylorus 105 to the ileo-caecal valve where it terminates in the large intestine (not shown). Generally, the small intestine 106 includes three sections: (i) the duodenum 108; (ii) the jejunum 118; and (iii) the ileum (not shown they are continuous and should be noted). The first 30cm (twelve-inch) section of the small intestine 106, the duodenum 108, is the shortest, widest and most fixed part of the small intestine 106.

The duodenum 108, in turn, includes four sections: (i) superior; (ii) descending; (iii) transverse; and (iv) ascending, which typically form a U-shape. The superior section is about 5 cm (two inches) long and ends at the neck of the gall bladder (not shown). The descending section is about 7.5 to 10 cm (three to four inches) long and includes, a nipple-shaped structure, referred to as the papilla of vater 110 through which pancreatic juice from the pancreas and bile produced by the liver and stored by the gall bladder, enter the duodenum. The pancreatic juice flows from the pancreas to the papilla of vater 110, through the pancreatic duct 112. Similarly, bile flows from the gall bladder to the papilla of vater 110, through the bile duct 114.

Both ducts 112,114 combine, as illustrated, before the papilla of vater 110, the anatomy of which is described in more detail below. The pancreatic juice contains enzymes essential to protein digestion; whereas, bile dissolves the products of fat digestion. Finally, the ascending section is about two inches long and forms the duodenal-jejunal flexure 116 where it joins the jejunum 118, the next section of the small intestine. The duodenal-jejunal flexure 116 is fixed to the ligament of Treitz 120 (musculus supensionus duodeni). Thus, the juices naturally secreted into the duodenum further break down the partially digested food into particles small enough to be absorbed by the body. The digestive system 100 is described in numerous texts, such as Gray's Anatomy ("Anatomy of the Human Body," by Henry Gray), and "human Physiology," Vander, 3rd ed, McGraw Hill, 1980.

Also illustrated in FIG.1 is an exemplary embodiment of an implantable enzyme sleeve 122. The sleeve 122 is generally anchored in alignment with a short dilated tube below where the common bile duct and the pancreatic duct join to ensure that both bile from the common bile duct and pancreatic enzymes from the pancreatic duct are collected into the sleeve 122. The short dilated tube is named the hepatopancreatic ampulla and also referred to as the ampulla of vater 124. The ampulla of vater 124 opens into the duodenum 108 through the major duodenal papilla (papilla of vater 110).

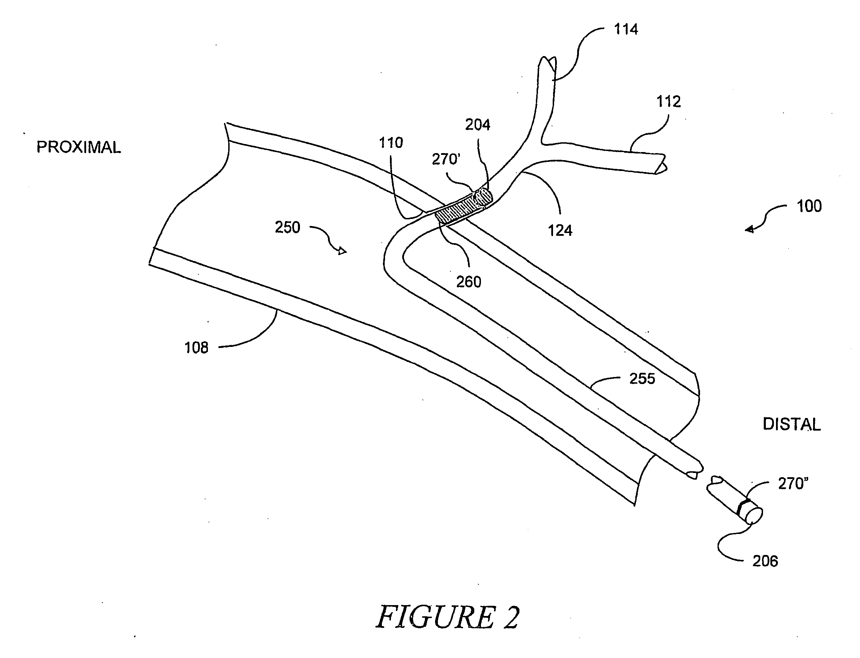

Referring now to FIG. 2, a sectional view of a portion of the digestive tract 100 is illustrated including a proximal end of an exemplary implanted enzyme sleeve 250. The enzyme sleeve 250 includes an elongated, open-ended, flexible sleeve or tube 255 having a first, proximal opening 204 and a second, distal opening 206. The sleeve 255 defines an interior lumen extending from the first, proximal opening 204 to the second, distal opening 206 for transporting the digestive enzymes secreted through the ampulla of vater 124. The surface of the passageway (the interior surface of the implant device 250) is preferably smooth to enable the enzymes to easily pass through. The exterior surface of the implant device 250 can also be smooth to prevent tissue in-growth, to be non-irritating to the bowel, and to allow chyme to pass through the intestine, unimpeded by the presence of the implant device 250.

According to the invention an anchor 260 is attached to the proximal end of the implant device 250 to secure the implant device in relation to the gastrointestinal tract 100. The anchor 260 is used to secure the proximal end of the device 250 to the ampulla of vater 124, such that when implanted, the anchor 260 resides at least partially within the ampulla of vater 124. In some embodiments, the anchor 260 can reside completely within the ampulla of vater 124 to reduce the possibility of irritation to the intestine. In other embodiments, the proximal end of the sleeve is secured in alignment with the papilla of vater 110 using sutures, surgical staples, an adhesive, combinations of these and any other suitable securing means. Additionally, a proximal end of the device 250 can be inserted through the papilla of vater 110, and secured within the ampulla of vater 124 using the anchor 260.

A strain relief element (not shown) can be optionally combined with the device 250 to prohibit kinking, or pinching of the elongated sleeve 255 in the vicinity of the papilla of vater 110. The strain relief element can include a supporting structure, such as a wire, coil, and/or struts configured to hold the sleeve 255 open. The supporting structure can be attached to the exterior of the sleeve 255, inserted within the interior of the sleeve 255, and/or placed between two layers of the sleeve 255. Alternatively, the strain relief element can include a separate material attached to the sleeve 255 in the vicinity of the papilla of vater 110. For example, a rigid, or semi-rigid tube segment (e.g. an elbow) can be coupled between the proximal end of the sleeve 255 and the anchor 260 providing the desired strain relief.

The distal end of the device 250 is placed into the duodenum 108 and extended distally from the papilla of vater 124, residing within the interior of the gastrointestinal tract 100. The implant device 250 terminates at a predetermined location within the intestine, generally determined by the length of the device 250. Typically, in human applications, the overall length of the device 250 ranges from about 30 cm to 1.5m (one foot to five feet). In some embodiments, the device can be up to 3m (10 feet) in length to extend into the ileum. The typical length of the device 250 is about 60 cm (2 feet) extending from the anchor 260 in the ampulla of vater 124 to below the ligament of Treitz 120. The length of the device 250 is generally selected to bypass the duodenum 108 and at least a portion of the jejunum 118. However, devices 250 of various lengths can be used to adjust the amount of absorption. For example, the length of the device 250 can be increased to further decrease absorption by bypassing a longer section of the jejunum 108. Additionally, the length of the device 250 can be variable and dependent on the patient's Body Mass Index (BMI). The diameter of the device is generally between about 5 and about 10 mm.

The sleeve material is preferably thin and conformable so that it collapses in the intestine to a small volume to minimize bowel irritability. In some embodiments, the thin-walled sleeve 255 is naturally in a collapsed state and is opened only by pressure from digestive enzymes within the sleeve 255. Further, the sleeve material preferably has a low coefficient of friction (e.g. less than about 0.20) so that enzymes slide easily through it and the bowel and chyme slide easily around it. Further, the sleeve material is preferably formed using a material having a low permeability to fluids, so that the enzymes touch neither the chyme, nor the bowel wall over the length of the sleeve 255. Thus, as the digestive enzymes are isolated within the sleeve, they do not significantly breakdown the chyme. Still further, the sleeve material is preferably biologically inert, impervious to digestive fluids, and non-irritating to the tissues.

In some embodiments, the sleeve material having the above-recited properties is formed using expanded polytetrafluoroethylene (ePTFE) with a wall thickness of about 0.127 mm (0.005 inch) with an internodal distance of less than about 5 microns. Notably, ePTFE is hydrophobic, yet slightly porous. The very small pores may become clogged over time. The porosity can be reduced by selectively coating the material on the inside, and/or the outside, and/or in the pores with dilute solutions of a sealant material, such as silicone or polyurethane.

In other embodiments, the sleeve 255 can be formed using a thin film of TEFLON® (e.g., PTFE, or FEP, or a combination thereof), polypropylene or polyethylene. For example, the film can have a wall thickness of less than about 0.025 mm (0.001 inch). The sleeve material must be sufficiently thin and pliable to permit peristalsis to propel the fluids inside the tube.

The anchor 260 is generally a cylindrical structure that defines an interior lumen and configured to communicate with the ampulla of vater 124. Thus, the anchor 260 has an interior surface in communication with the digestive enzymes, and an exterior surface in communication with the body. The external diameter of the anchor 260 generally depends upon the diameter of the ampulla of vater 124, the anchor 260 being sized to fit therein. Thus, in human applications the external diameter of the anchor 260 is typically between about 5 and about 10 mm, being preferably between about 8 and about 10 mm, based on human anatomy variations.

The length of the anchor 260 measured along its central axis is selected based on its application. For example, for an anchor inserted within the ampulla of vater 124 the length extends from the entry point at the interior surface of the duodenum 108 (i.e., the papilla of vater 110) into the ampulla of vater 124. In some applications, a maximum length is determined, such that the anchor does not extend beyond the point at which the bile duct 114 and the pancreatic duct 112 merge. In some embodiments, the length of the anchor 260 is selected to allow at least a proximal part of the anchor 260 to extend into duodenum 108. In other embodiments, a shorter length is selected for the anchor 260, such that the anchor 260, when positioned within the ampulla of vater 124, does not extend into the duodenum 108. Thus, the anchor 260 generally has an overall length from about 1 to about 5 centimeters. Notably, the dimensions for the anchor 260 recited herein are similar to the dimensions of a biliary stent, commonly used within the bile duct 114.

The anchor 260 can be formed as a collapsible and self-expanding device, such as a collapsible, self-expanding tube, or stent. Thus, the anchor 260 can be securedly attached to the ampulla of vater 124 using an interference fit. In addition, the anchor 260 can be attached to the gastrointestinal tract 100 using sutures, staples, adhesive, a combination of these, or other suitable means. Preferably, the anchor 260 is removably attached, such that the device 250 can be implanted and removed as required with relative ease. For example, the anchor 260 can be formed from a shaped memory material, such as a nickel-titanium (Ni-Ti) compound. One such Ni-Ti alloy is commonly referred to as nitinol, that can be formed into an anchor. The nitinol anchor can be similar in design to the pyloric anchoring device described in U. S. Patent publications US-A-7025791 and US-A-2007/027548.

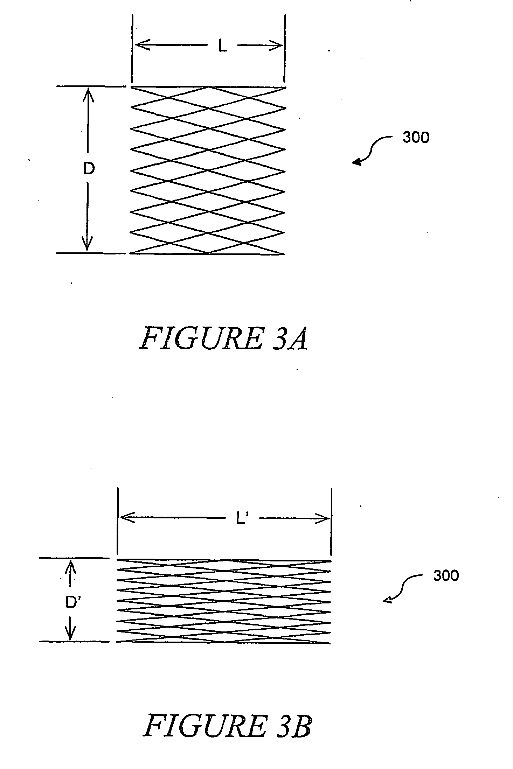

An exemplary tubular anchor 300 can be formed using a network of struts as shown in an expanded configurations in FIG. 3A and in a collapsed configuration in FIG. 3B. Thus, the anchor 300 can be formed from interconnecting struts that form a mesh (e.g., a network of struts). For example, the struts can form a mesh having diamond spaced openings, as illustrated, that are sufficiently flexible to allow the stent to be collapsed inside a delivery catheter and have sufficient elasticity to expand to secure the anchor to the papilla of vater once the catheter is withdrawn. As described above, the struts can be formed from a shape memory material, such as nitinol. Thus, in some embodiments, the anchor is compliant. Alternatively, the anchor can be constructed of a rigid material, such as a rigid metal or plastic. The rigid material can be expandable to facilitate insertion into the body, but once expanded, can retain its expanded shape. Alternatively, the anchor can be non- expandable.

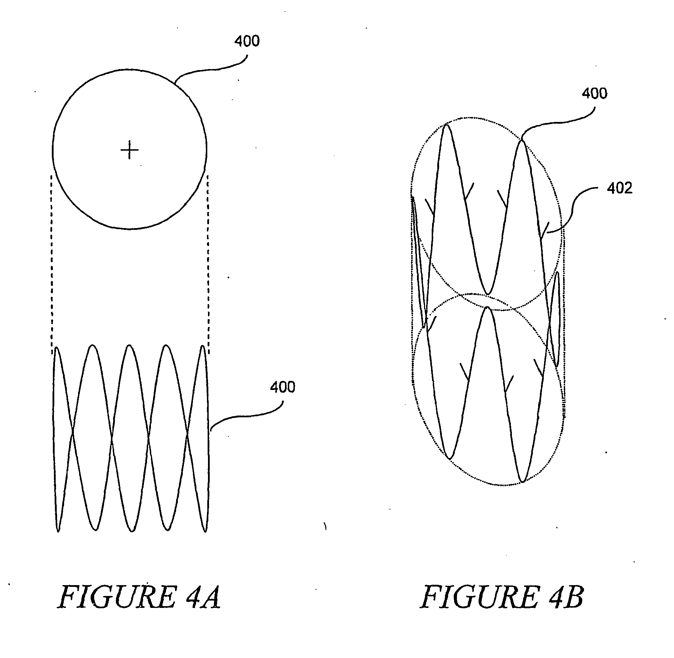

The tubular anchor 330 can also be formed using a single, continuous supporting member, such as the "wave" shape illustrated in FIGS. 4A and 4B. The wave shape of the supporting member allows for compression in the radial direction to facilitate insertion and/or removal. As shown in FIG. 4B, the wave anchor can include one or more barbs 402 configured for securing the anchor in its installed position. For example, the wave anchor can be similar in design to the anchoring device described in U. S. Application US-A-2005/0125020.

As described above in relation to FIG. 2, the anchor 260 can be secured in the ampulla of vater 124, where the common bile duct 114 and the pancreatic duct 112 join and extend through the papilla of vater 110 into the duodenum 108. As described above in relation to the sleeve 255, the anchor 260 can also be selectively coated with a different material having properties adapted for a specific application.

For example, the anchor 260 can be coated with PTFE material to limit in-growth of tissue making it easier to remove the device. Further, the anchor 260 can also be coated with a pharmaceutically-active compound, such as an anti-inflammatory, or antirejection drug. This drug could also be incorporated into the sleeve material. The means for delivering drugs can be similar in design to the drug-delivery means described in U.S. Application US-A-2005/0125020.

The anchor 260, in turn, can be fastened to the proximal end of the flexible sleeve 255 using a number of fastening techniques. For example, the anchor 260 can be attached to the sleeve 255 using a chemical fastener, such as an adhesive, and/or or thermal bonding. Additionally, the anchor 260 can be attached to the sleeve 255 using mechanical fastener, such as a suture, a staple, or any other suitable means including combinations of mechanical and chemical fasteners. Preferably, the joint formed between the anchor 260 and the sleeve 255 is fluid tight, to prohibit, or at least limit, digestive enzymes leaking and mixing with the chyme near the proximal end of the sleeve 255.

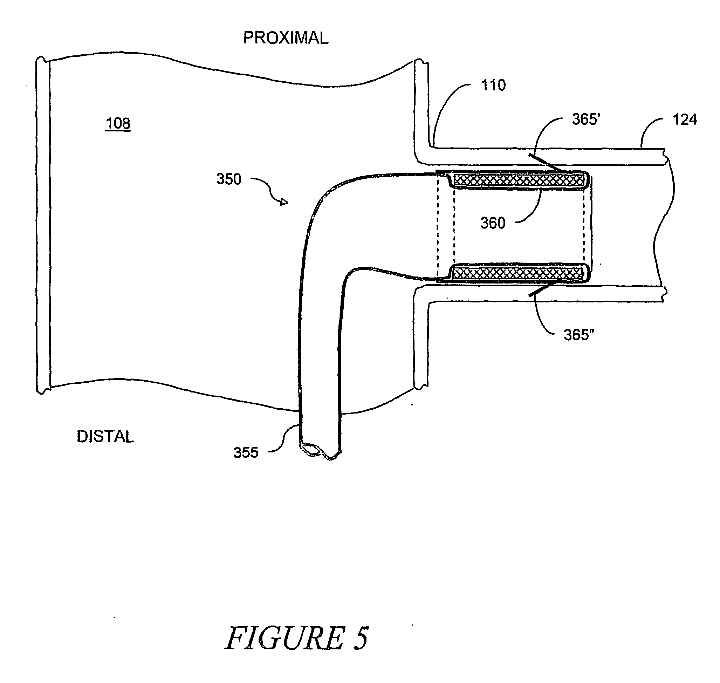

In still further embodiments, the anchor 260 is housed between two layers of the proximal end of the sleeve 255. For example, referring now to FIG. 5, an anchor 360 is placed between two layers of the proximal end of the sleeve 355. As shown, the proximal end of the sleeve can extend through the lumen of the cylindrical anchor 360, beyond the proximal end of the anchor 360, then fold back distally, along the outside surface of the anchor 360. In some embodiments, the sleeve can extend beyond the full length of the anchor 360, such that the anchor is completely enclosed within the sleeve material.

Optionally, the anchor 360 can include one or more mechanical fasteners, such as barbs. As shown, the anchor 360 includes a number of unidirectional barbs 365', 365" (generally 365), each barb attached at one end to the anchor 360 and extending outward, in a direction that is substantially parallel to the central axis of the anchor 360. The other end of each barb 365 extends outward from the exterior of the anchor 360, such that the barb 365 forms an angle with the surface of the anchor 360, as shown. As peristalsis tends to drag the implant device 350 distally along the gastrointestinal tract 100, the barbs 365 can be aligned within the ampulla of vater 124 to point distally, as shown. Thus, as the barbs 365 penetrate the surrounding tissue, they secure the proximal end of the implant 350 to the surrounding tissue. In other embodiments, the barbs 365 can be aligned in multiple directions, such as opposing directions to further secure the proximal end of the implant 350 under changing forces within the gastrointestinal tract 100.

In one embodiment, the sleeve 355 includes two layers of material at least at the proximal end. A first outer layer covers the exterior of the anchor 360 as described above. The second inner layer covers the interior surface of the anchor 360. The barbs 365 protrude from the exterior surface of the anchor 360 through the first outer layer of the sleeve 355. The holes in the first outer layer through which the barbs 365 protrude can be filled with an impervious material such as silicone or urethane to seal the sleeve 355. The diameter of the sleeve 355 is selected such that the first outer layer of the sleeve 355 fits over the anchor 360.

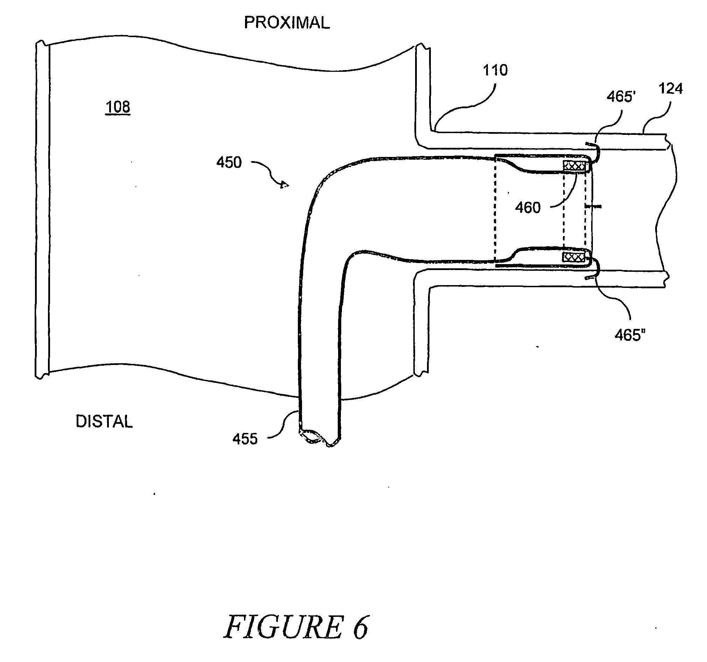

An alternative means of anchoring the device is illustrated in FIG. 6. An anchoring ring 460 formed from a flexible ring, such as a silicone ring, is combined with one or more retractable staples 465', 465", 465"' (generally 465) to secure the anchor 460 within the ampulla of vater 124. Notably, although the diameter of the anchoring ring 460 is still between about 5 and about 10 mm, preferably between about 8 and about 10 mm, the length of the anchoring ring 460 measured along its axis can be substantially less than the previously described anchors 260, 360. As the device 460 does not rely on an interference fit alone to hold it in place, it does not require contact with the bodily tissue over a substantial area. Rather, the anchoring ring 460 is attached to the proximal end of an elongated, flexible sleeve 455 by any of the above-described techniques, then attached to the surrounding tissue using a mechanical fastening means, such as removable staples. For example, the anchoring ring 460 can be similar in design to the anchoring ring described in U.S. Patent Application US-A- 2007/027548.

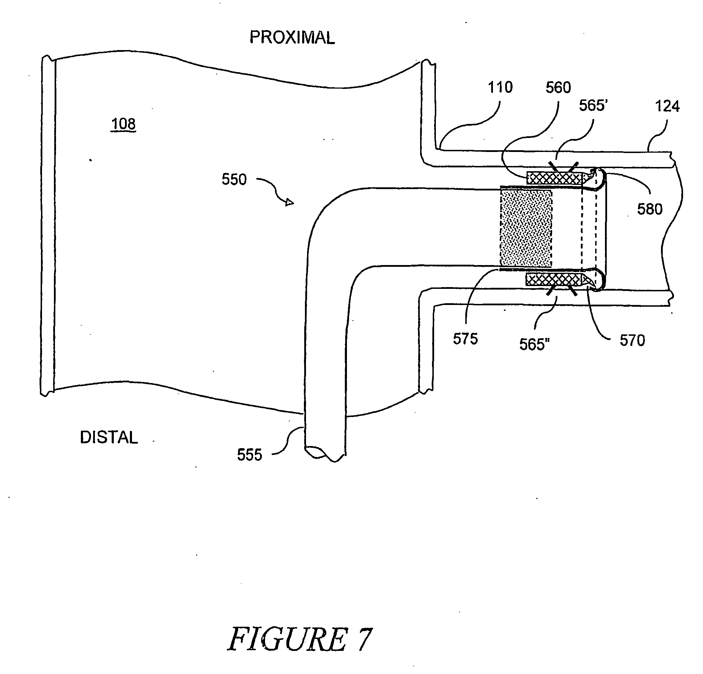

In an alternative embodiment, referring now to FIG. 7, an enzyme sleeve 550 is proximally anchored within the ampulla of vater 124 using a two-piece system. The two-piece system includes a non-removable element 570 designed to remain in-place within the body, and a removable element 575 designed to attach to a proximal end of the sleeve 555 for removably coupling the proximal end of the sleeve 555 to the non-removable element. The non-removable element 570, or stent, is first placed implanted in the ampulla of vater 124. For example, a non-removable element, or fixed anchor 570 can be provided with porosity to encourage tissue growth into it. Advantageously, the fixed anchor 570 includes a feature adapted for coupling to the removable element 575. Likewise, the removable element 575 includes a feature 580 that is complementary to the feature of the fixed anchor 570 to facilitate the coupling therebetween. In one example, the fixed anchor 570 includes a proximal rim. The removable element 575 at the proximal end of the sleeve 555 includes a complementary feature 580 designed to grasp the rim of the fixed anchor 570. For example, the removable element 575 includes lip 580, or one or more hooks (not shown) at its distal end configured to couple to the fixed anchor 570, thereby holding the sleeve 555 in place. The sleeve 555 can be removed by disengaging the removable element 575 from the fixed anchor 570, collapsing the removable element 575, and pulling it through the fixed anchor 570. Other embodiments can include hook-and-loop, and notch-and-detent, configurations for removably attaching the removable anchor 580 to the fixed anchor 570.

The implantable enzyme sleeve 250 is preferably designed for endoscopic implantation and removal. Advantageously, the covered anchor 260 can be collapsed into a sheath having a diameter less than about 6,35 mm (0.25 inch) to enable endoscopic delivery. Covering the exterior surface of the anchor 260 with the first outer layer of the sleeve 255 permits endoscopic removal of the implant device 250 by preventing tissue in-growth on the exterior surface of the anchor 260.

A catheter system such as the catheter described in U.S. Patent Application US-A-2007/027548 can be used. For example, a catheter follows a guide wire through the esophagus, through the stomach 102, through the pylorus portion of the stomach 105, and into the duodenum 108. The expandable anchor 260 is then endoscopically inserted in a collapsed state into the ampulla of vater 124 and allowed to expand to its natural shape or simply pushed in place. When expanded, the anchor 260 secures the proximal end of the sleeve. The distal end of the sleeve is then extended through the intestine to its fully extended length. This procedure is a substantially less invasive alternative to surgery for the treatment of obesity and morbid obesity and also provides a new treatment approach for Type-2 diabetes. Additionally, as the device can be selectively removed, it offers patients a reversible option that can be tailored to a patient's changing needs. Alternatively, the enzyme sleeve 250 can be percutaneously implanted, for example using a laparoscope.

Endoscopic insertion of the device 250 can be accomplished using a specially configured scope, such as an Endoscopic Retrograde CholangioPancreatography (ERCP) scope. ERCP scopes are adapted to insert stents from the intestine into the bile and/or pancreatic ducts. Thus the anchor 260 can be secured to the ampulla of vater 124 using the ERCP scope. Once secured, the distal portion of the sleeve 255 can be fully extended downstream using a catheter, relying on natural peristalsis, or using a combination of a catheter placement and peristalsis. Alternatively, the distal portion of the sleeve 255 can be first placed at its intended position within the intestine using a catheter. Then the proximal end of the device 250 can be subsequently anchored to the ampulla of vater 124 using a scope, such as the ERCP scope.

Referring again to FIG. 2, markings 270', 270" (generally 270) can be added to the exterior surface of the sleeve 255 to detect on a fluoroscopic image the position and orientation of the sleeve and whether the sleeve is twisted. For example, a stripe can be painted down the length of the device 250 using tantulum impregnated ink, or tantulum bands can be bonded to the interior surface of the device 250.

In operation, the elongated flexible sleeve 250 provides weight loss mechanisms by providing negative feedback, reduced fat digestion and reduced appetite. The reduced fat digestion occurs because the sleeve 250 delays the mixing of bile and pancreatic juices with chyme from the stomach until after the chyme leaves the sleeve. The reduced appetite may occur because the reduced digestion in the duodenum caused by the sleeve 255 may reduce hormonal release from the duodenum. Also, it is believed that the presence of undigested chyme in the ileum triggers release of peptide YY (PYY) which is believed to reduce appetite.

After the digestive enzymes have passed through the sleeve 255, the sleeve 255 becomes extremely thin and floppy, permitting the sleeve 255 to contour to the inner walls of the intestine 106. The sleeve 255 is non-compliant and drapes away from the intestinal walls. The normal peristalsis of the bowel used to propel the chyme through the intestines 106, also propels the digestive enzymes through the sleeve 255.

While this invention has been particularly shown and described with references to preferred embodiments thereof, it will be understood by those skilled in the art that various changes in form and details may be made therein without departing from the scope of the invention encompassed by the appended claims.

## Claims

1. A gastrointestinal implant device comprising:
an elongated tube (255) defining a central lumen, the tube (255) being open at both ends (204,206), and adapted to extend into the duodenum; **characterized by**
an anchor (260) for coupling the proximal end (204) of the tube (255) in alignment with the hepatopancreatic ampulla, wherein the anchor (260) is adapted to be positioned within the hepatopancreatic ampulla, the tube (255) being able to pass digestive enzymes from the hepatopancreatic ampulla into a distal portion of the gastrointestinal tract.

2. The gastrointestinal implant device of claim 1, wherein the tube (255) comprises a flexible sleeve.

3. The gastrointestinal implant device of claim 1, wherein the distal portion of the gastrointestinal tract is the distal jejunum.

4. The gastrointestinal implant device of claim 1, wherein the flexible sleeve (255) is formed of expanded polytetrafluoroethylene (ePTFE).

5. The gastrointestinal implant device of claim 1, wherein the flexible sleeve (255) is formed of polyethylene.

6. The gastrointestinal implant device of claim 1, wherein the flexible sleeve (255) comprises a coating.

7. The gastrointestinal implant device of claim 6, wherein the coating is a polyurethane-based coating.

8. The gastrointestinal implant device of claim 6, wherein the coating is a silicone-based coating.

9. The gastrointestinal implant device of claim 1, wherein the sleeve material has a coefficient of friction of less than about 0.2.

10. The gastrointestinal implant device of claim 1, wherein the anchor (260) has an external diameter between 5 and 10 millimeters.

11. The gastrointestinal implant device of claim 10, wherein the anchor (260) has an external diameter between 8 and 10 millimeters.

12. The gastrointestinal implant device of claim 1, wherein the anchor (260) has a length between 1 and 5 centimeters.

13. The gastrointestinal implant device of claim 1, wherein the anchor (260) is collapsible.

14. The gastrointestinal implant device of claim 13, wherein the anchor (260) is formed of shape memory material.

15. The gastrointestinal implant device of claim 14, wherein the shape memory material comprises a nickel-titanium (Ni-Ti) alloy.

16. The gastrointestinal implant device of claim 1, wherein the anchor (260) comprises a stent.

17. The gastrointestinal implant device of claim 1, wherein the anchor (260) is at least partially covered by a proximal portion of the flexible sleeve (255).

18. The gastrointestinal implant device of claim 1, wherein the anchor (330) further comprises barbs (402) extending from the exterior surface of the anchor (330), the barbs (402) configured for securing the proximal portion of the sleeve (255) in the hepatopancreatic ampulla.

19. The gastrointestinal implant device of claim 18, wherein the barbs (402) are configured to penetrate bodily tissue.

20. The gastrointestinal implant device of claim 18, wherein the barbs (402) are substantially bi-directional, extending outward, in opposing directions that are substantially parallel to the central axis of the proximal end of the flexible sleeve (255).

21. The gastrointestinal implant device of claim 1, wherein the anchor further comprises:
a non-removable element (570) adapted to be securedly coupled in the hepatopancreatic ampulla; and
a removable element (575) coupled to the proximal end of the sleeve (555), the removable element (575) removably coupled to the non-removable element (570) for removably securing, in use, the proximal end of the sleeve (555) in the hepatopancreatic ampulla.

22. The gastrointestinal implant device of claim 21, wherein the non-removable element (570) comprises barbs extending from its exterior surface for securing it in the hepatopancreatic ampulla.

23. The gastrointestinal implant device of claim 21, wherein the non-removable element (570) comprises a feature adapted for coupling the removable element (575).

24. The gastrointestinal implant device of claim 1, wherein the anchor (260) further comprises an annular element having retractable staples, the staples coupled to bodily tissue, when engaged.

## Patentansprüche

1. Magen-Darm-Implantatanordnung, die aufweist:
einen langgestreckten Schlauch (255), der ein in der Mitte angeordnetes Lumen abgrenzt, wobei der Schlauch (255) an beiden Enden (204, 206) offen ist und angepasst ist sich in den Zwölffingerdarm zu erstrecken;
**gekennzeichnet durch**
eine Verankerung (260) zur Kopplung des proximalen Endes (204) des Schlauchs (255) in Ausrichtung mit der Hepatopankreasampulle, wobei die Verankerung (260) angepasst ist in der Hepatopankreasampulle angeordnet zu werden, wobei der Schlauch (255) dazu fähig ist Verdauungsenzyme von der Hepatopankreasampulle in einen distalen Teil des Magen-Darm-Trakts zu leiten.

2. Magen-Darm-Implantatanordnung nach Anspruch 1, wobei der Schlauch (255) eine flexible Hülle aufweist.

3. Magen-Darm-Implantatanordnung nach Anspruch 1, wobei der distale Teil des Magen-Darm-Trakts das distale Jejunum ist.

4. Magen-Darm-Implantatanordnung nach Anspruch 1, wobei die flexible Hülle (255) aus erweitertes Polytetrafluoroäthylen ausgeformt ist.

5. Magen-Darm-Implantatanordnung nach Anspruch 1, wobei die flexible Hülle (255) aus Polyäthylen ausgeformt ist.

6. Magen-Darm-Implantatanordnung nach Anspruch 1, wobei die flexible Hülle (255) eine Beschichtung aufweist.

7. Magen-Darm-Implantatanordnung nach Anspruch 6, wobei die Beschichtung eine auf Polyurethan basierende Beschichtung ist.

8. Magen-Darm-Implantatanordnung nach Anspruch 6, wobei die Beschichtung eine auf Silikon basierende Beschichtung ist.

9. Magen-Darm-Implantatanordnung nach Anspruch 1, wobei das Material der Hülle einen Reibungskoeffizienten von weniger als etwa 0,2 aufweist.

10. Magen-Darm-Implantatanordnung nach Anspruch 1, wobei die Verankerung (260) einen Außendurchmesser zwischen 5 und 10 Millimetern aufweist.

11. Magen-Darm-Implantatanordnung nach Anspruch 10, wobei die Verankerung (260) einen Außendurchmesser zwischen 8 und 10 Millimetern aufweist.

12. Magen-Darm-Implantatanordnung nach Anspruch 1, wobei die Verankerung (260) eine Länge zwischen 1 und 5 Zentimeter aufweist.

13. Magen-Darm-Implantatanordnung nach Anspruch 1, wobei die Verankerung (260) zusammenklappbar ist.

14. Magen-Darm-Implantatanordnung nach Anspruch 13, wobei die Verankerung (260) aus Formgedächtnismaterial ausgeformt ist.

15. Magen-Darm-Implantatanordnung nach Anspruch 14, wobei das Formgedächtnismaterial eine Nickel-Titan- (Ni-Ti) Legierung aufweist.

16. Magen-Darm-Implantatanordnung nach Anspruch 1, wobei die Verankerung (260) eine endoluminale Gefäßprothese aufweist.

17. Magen-Darm-Implantatanordnung nach Anspruch 1, wobei die Verankerung (260) zumindest teilweise durch einen proximalen Teil der flexiblen Hülle (255) bedeckt ist.

18. Magen-Darm-Implantatanordnung nach Anspruch 1, wobei die Verankerung (330) weiterhin Spitzen (402) aufweist, die sich von der äußeren Oberfläche der Verankerung (330) erstrecken, wobei die Spitzen (402) ausgestaltet sind, um den proximalen Teil der Hülle (255) in der Hepatopankreasampulle zu fixieren.

19. Magen-Darm-Implantatanordnung nach Anspruch 18, wobei die Spitzen (402) ausgestaltet sind, um Körpergewebe zu durchdringen.

20. Magen-Darm-Implantatanordnung nach Anspruch 18, wobei die Spitzen (402) im Wesentlichen bidirektional sind und sich in entgegen gesetzte Richtungen nach außen erstrecken, die im Wesentlichen parallel zur Mittelachse des proximalen Endes der flexiblen Hülle (255) sind.

21. Magen-Darm-Implantatanordnung nach Anspruch 1, wobei die Verankerung weiterhin aufweist:
ein nicht entfernbares Element (570) das angepasst ist fest in der Hepatopankreasampulle verankert zu werden; und
ein entfernbares Element (575), gekoppelt an das proximale Ende der Hülle (255), wobei das entfernbare Element (575) entfernbar mit dem nicht entfernbaren Element (570) gekoppelt ist, um das proximale Ende der Hülle (255) während der Verwendung entfernbar in der Hepatopankreasampulle zu fixieren.

22. Magen-Darm-Implantatanordnung nach Anspruch 21, wobei das nicht entfernbare Element (570) Spitzen aufweist, die sich von seiner äußeren Oberfläche erstrecken um es in der Hepatopankreasampulle zu fixieren.

23. Magen-Darm-Implantatanordnung nach Anspruch 21, wobei das nicht entfernbare Element (570) eine Eigenschaft aufweist die angepasst ist das entfernbare Element (575) zu koppeln.

24. Magen-Darm-Implantatanordnung nach Anspruch 1, wobei die Verankerung (260) weiterhin ein ringförmiges Element aufweist, das einziehbare Krampen aufweist, wobei die Krampen mit Körpergewebe verbunden sind, wenn sie eingreifen.

## Revendications

1. Dispositif d'implant gastro-intestinal comprenant :
un tube allongé (255) définissant une lumière centrale, le tube (255) étant ouvert aux deux extrémités (204, 206), et adapté pour s'étendre dans le duodénum ; **caractérisé par**
un ancrage (260) pour le couplage de l'extrémité proximale (204) du tube (255) en alignement avec l'ampoule hépatopancréatique, où l'ancrage (260) est adapté pour être positionné dans l'ampoule hépatopancréatique, le tube (255) étant capable de faire passer des enzymes digestives de l'ampoule hépatopancréatique dans une partie distale du tractus gastro-intestinal.

2. Dispositif d'implant gastro-intestinal selon la revendication 1, dans lequel le tube (255) comprend un manchon flexible.

3. Dispositif d'implant gastro-intestinal selon la revendication 1, dans lequel la partie distale du tractus gastro-intestinal est le jéjunum distal.

4. Dispositif d'implant gastro-intestinal selon la revendication 1, dans lequel le manchon flexible (255) est formé de polytétrafluoroéthylène augmenté.

5. Dispositif d'implant gastro-intestinal selon la revendication 1, dans lequel le manchon flexible (255) est formé de polyéthylène.

6. Dispositif d'implant gastro-intestinal selon la revendication 1, dans lequel le manchon flexible (255) comprend un revêtement.

7. Dispositif d'implant gastro-intestinal selon la revendication 6, dans lequel le revêtement est un revêtement à base de polyuréthane.

8. Dispositif d'implant gastro-intestinal selon la revendication 6, dans lequel le revêtement est un revêtement à base de silicone.

9. Dispositif d'implant gastro-intestinal selon la revendication 1, dans lequel le matériau du manchon possède un coefficient de friction inférieur à environ 0,2.

10. Dispositif d'implant gastro-intestinal selon la revendication 1, dans lequel l'ancrage (260) possède un diamètre externe compris entre 5 et 10 mm.

11. Dispositif d'implant gastro-intestinal selon la revendication 10, dans lequel l'ancrage (260) possède un diamètre externe compris entre 8 et 10 mm.

12. Dispositif d'implant gastro-intestinal selon la revendication 1, dans lequel l'ancrage (260) possède une longueur comprise entre 1 et 5 cm.

13. Dispositif d'implant gastro-intestinal selon la revendication 1, dans lequel l'ancrage (260) est compressible.

14. Dispositif d'implant gastro-intestinal selon la revendication 13, dans lequel l'ancrage (260) est formé d'un matériau à mémoire de forme.

15. Dispositif d'implant gastro-intestinal selon la revendication 14, dans lequel le matériau à mémoire de forme comprend un alliage nickel-titane (Ni-Ti).

16. Dispositif d'implant gastro-intestinal selon la revendication 1, dans lequel l'ancrage (260) comprend une endoprothèse vasculaire.

17. Dispositif d'implant gastro-intestinal selon la revendication 1, dans lequel l'ancrage (260) est revêtu au moins partiellement par une partie proximale du manchon flexible (255).

18. Dispositif d'implant gastro-intestinal selon la revendication 1, dans lequel l'ancrage (330) comprend en outre des barbes (402) s'étendant depuis la surface extérieure de l'ancrage (330), les barbes (402) étant configurées pour fixer la partie proximale du manchon (255) dans l'ampoule hépatopancréatique.

19. Dispositif d'implant gastro-intestinal selon la revendication 18, dans lequel les barbes (402) sont configurées pour pénétrer les tissus corporels.

20. Dispositif d'implant gastro-intestinal selon la revendication 18, dans lequel les barbes (402) sont sensiblement bidirectionnelles, s'étendant vers l'extérieur, dans des directions opposées qui sont sensiblement parallèles à l'axe central de l'extrémité proximale du manchon flexible (255).

21. Dispositif d'implant gastro-intestinal selon la revendication 1, dans lequel l'ancrage comprend en outre :
un élément non amovible (570) adapté pour être couplé de manière fixe dans l'ampoule hépatopancréatique ; et
un élément amovible (575) couplé à l'extrémité proximale du manchon (555), l'élément amovible (575) étant couplé de manière amovible à l'élément non amovible (570) pour fixer de manière amovible, en utilisation, l'extrémité proximale du manchon (555) dans l'ampoule hépatopancréatique.

22. Dispositif d'implant gastro-intestinal selon la revendication 21, dans lequel l'élément non amovible (570) comprend des barbes s'étendant à partir de sa surface extérieure pour le fixer dans l'ampoule hépatopancréatique.

23. Dispositif d'implant gastro-intestinal selon la revendication 21, dans lequel l'élément non amovible (570) comprend une caractéristique adaptée pour le couplage de l'élément amovible (575).

24. Dispositif d'implant gastro-intestinal selon la revendication 1, dans lequel l'ancrage (260) comprend en outre un élément annulaire ayant des agrafes rétractiles, les agrafes étant couplées aux tissus corporels, quand elles sont mises en prise.
